# EUROPEAN PATENT APPLICATION

(11) **EP 3 318 280 A1**
(43) Date of publication of application: **09.05.2018**
(21) Application number: 16818049.5
(22) Date of filing: 30.06.2016
(51) Int. Cl.: A61K 47/14, A61K 9/06, A61K 31/444, A61K 47/34, A61P 27/02, A61P 27/06, A61P 31/04

(54) **DEPOT PREPARATION CONTAINING CITRIC ACID ESTER**

(30) Priority: 01.07.2015 JP 2015133040
(71) Applicant: Santen Pharmaceutical Co., Ltd, Osaka-shi, Osaka 533-8651 (JP)
(72) Inventor: MIYAZAKI, Tatsuya, Inukami-gun Shiga 522-0314 (JP); MASAKI, Kenji, Ikoma-shi Nara 630-0101 (JP); TAKAHASHI, Kyohei, Ikoma-shi Nara 630-0101 (JP); YAMADA, Kazuhito, Ikoma-shi Nara 630-0101 (JP)
(74) Representative: Müller-Boré & Partner Patentanwälte PartG mbB
(86) International application number: PCT/JP2016/069522
(87) International publication number: WO 2017/002941

(57) **Abstract**

To provide a depot formulation for which depot formation is easy, and is capable of maintaining the depot form for a long period as desired, and in the case of containing a drug, to provide a depot formulation that sustained-releases the drug over a long period after being administered into the body. The present invention relates to a depot formulation containing a trialkyl citrate and/or an acetyl trialkyl citrate, in which the alkyl groups possessed by each of the trialkyl citrate and the acetyl trialkyl citrate are the same or different, and have a carbon number of 3 to 5.

## Description

### TECHNICAL FIELD

The present invention relates to a depot formulation containing a trialkyl citrate and/or acetyl trialkyl citrate, in which the carbon number of the alkyl groups in the ester is 3 to 5.

The present invention further relates to the above-mentioned depot formulation containing a drug, and particularly relates to a depot formulation containing, as the drug, a compound represented by formula (1) or a salt thereof: 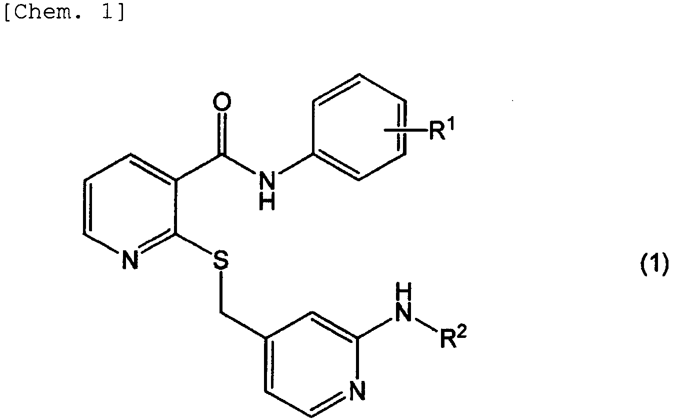 (In the formula,
R¹ denotes a halogen atom, hydroxyl group, C₁₋₆ alkyl group, C₁₋₆ alkyl group substituted by one or a plurality of halogen atoms, C₁₋₆ alkoxy group, or C₁₋₆ alkoxy group substituted by one or a plurality of halogen atoms; and
R² denotes a hydrogen atom, C₁₋₆ alkyl group, C₁₋₆ alkylcarbonxyl group, or C₁₋₆ alkylcarbonxyl group substituted by one or a plurality of hydroxyl groups).

The present invention particularly relates to a depot formulation containing isopropyl (6-{[4-(pyrazol-1-yl)benzyl](pyridin-3-ylsulfonyl)aminomethyl}pyridin-2-ylamino)acetate or a salt thereof as the above-mentioned drug.

### BACKGROUND ART

For example, an invasive medicine like an intravitreal injection is desired to be a formulation for which the drug is controlled released from the administered site after the drug is administered into the body, and that exhibits drug efficacy over a long period, from the viewpoint of the drug administrating burden on the patient, etc. As a means for realizing this, a depot formulation has been known that forms a depot at the site at which a drug is administered, and the drug is controlled released from this site.

Patent Document 1 describes the matter of exhibiting a drug sustained releasability in the case of a formulation of dexamethasone containing acetyl triethyl citrate (ATEC) and a polymer such as polylactic acid (PLA), compared to a preparation not including polymer. However, Patent Document 1 has no specific disclosure of a depot formulation containing an acetyl trialkyl citrate other than acetyl triethyl citrate or a depot formulation containing a trialkyl citrate, and does not disclose drug sustained releasability in the case of not containing a polymer such as PLA.

Although Patent Documents 2 and 3 disclose injectable formulations containing solvents and polymers such as polylactic acid (PLA), there is no specific disclosure of a depot formulation containing trialkyl citrate and/or acetyl trialkyl citrate, and they do not disclose drug sustained releasability in the case of these not containing a polymer such as PLA.
Patent Document 1: PCT International Publication No. WO2013/036309
Patent Document 2: PCT International Publication No. WO2005/048989
Patent Document 3: PCT International Publication No. WO2004/011054

### DISCLOSURE OF THE INVENTION

### Problems to be Solved by the Invention

However, there are cases where a formulation having higher drug sustained releasability than the above-mentioned conventional formulations containing acetyl triethyl citrate is desired.

Taking account of the above-mentioned situation, the problem of the present invention is to provide a depot formulation for which depot formation is easy, and is able to maintain the depot form for a long period as desired, and in the case of containing a drug, to provide a depot formulation that sustained-releases the drug over a long period after administered into the body.

### Means for Solving the Problems

As a result of conducting thorough research into additives, solvents, etc. for forming depots in order to solve the above-mentioned problems, the present inventors found that a depot formulation containing triaklyl citrate and/or acetyl trialkyl citrate, in which the carbon number of the alkyl groups in the ester is 3 to 5, readily forms a depot, and is capable of maintaining the depot form for a long period as desired, and in the case of containing a drug, sustained-releases the drug, thereby arriving at completion of the present invention.

More specifically, the present invention is related to the following.

According to a first aspect of the present invention, a depot formulation contains a trialkyl citrate and/or acetyl trialkyl citrate, in which alkyl groups possessed by each of the trialkyl citrate and the acetyl trialkyl citrate are the same or different, and have a number of carbon atoms of 3 to 5.

According to a second aspect of the present invention, in the depot formulation as described in the first aspect, the number of carbon atoms of the alkyl group is 4.

According to a third aspect of the present invention, in the depot formulation as described in the first aspect, the trialkyl citrate is tri-n-butyl citrate, and the acetyl trialkyl citrate is acetyl tri-n-butyl citrate.

According to a fourth aspect of the present invention, the depot formulation as described in any one of the first to third aspects further contains a drug.

According to a fifth aspect of the present invention, in the depot formulation as described in the fourth aspect, the drug is a compound represented by formula (1) or a salt thereof, 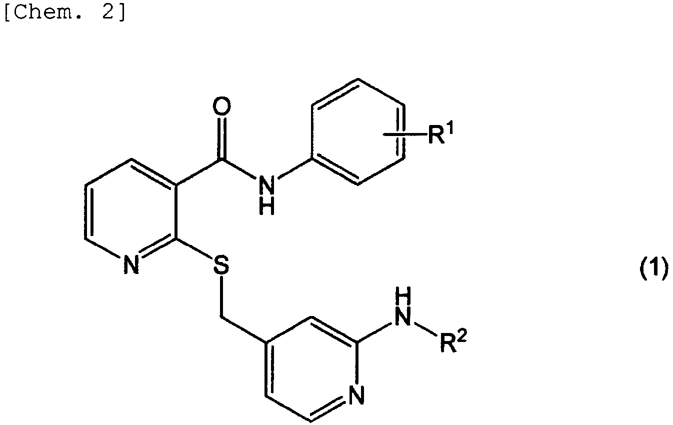 where, in the formula,
R¹ denotes a hydrogen atom, halogen atom, hydroxyl group, C₁₋₆ alkyl group, C₁₋₆ alkyl group substituted by one or a plurality of halogen atoms, C₁₋₆ alkoxy group or C₁₋₆ alkoxy group substituted by one or a plurality of halogen atoms; and
R² denotes a hydrogen atom, C₁₋₆ alkyl group, C₁₋₆ alkylcarbonyl group or C₁₋₆ alkylcarbonyl group substituted by one or a plurality of hydroxyl groups.

According to a sixth aspect of the present invention, in the depot formulation as described in the fifth aspect, in formula (1),
R¹ denotes a C₁₋₆ alkoxy group or a C₁₋₆ alkoxy group substituted by one or a plurality of halogen atoms, and
R² denotes a C₁₋₆ alkylcarbonyl group or a C₁₋₆ alkylcarbonyl group substituted by one or a plurality of hydroxy groups.

According to a seventh aspect of the present invention, in the depot formulation as described in the fifth aspect, in formula (1),
R¹ denotes a C₁₋₆ alkoxy group substituted by one or a plurality of halogen atoms, and
R² denotes a C₁₋₆ alkylcarbonyl group substituted by one or a plurality of hydroxy groups.

According to an eighth aspect of the present invention, in the depot formulation as described in the fifth aspect, the compound represented by formula (1) is 2-[[[2-[(hydroxyacetyl)amino]-4-pyridinyl]methyl]thio]-N-[4-(trifluoromethoxy)phenyl]-3-pyridinecarboxamide or a salt thereof.

According to a ninth aspect of the present invention, in the depot formulation as described in the fourth aspect, the drug is isopropyl (6-{[4-(pyrazol-1-yl)benzyl](pyridin-3-ylsulfonyl)aminomethyl}pyridin-2-ylamino)acetate or a salt thereof.

According to a tenth aspect of the present invention, in the depot formulation as described in the fourth aspect, the drug is nepafenac, dexamethasone, indomethacin, diclofenac sodium, levofloxacin, INCB28050, ciclosporin A, timolol maleate, fluocinolone acetonide, triamcinolone acetonide, budesonide, olopatadine, latanoprost, isopropyl (6-{[4-(pyrazol-1-yl)benzyl](pyridin-3-ylsulfonyl)aminomethyl}pyridin-2-ylamino)acetate, 2-[[[2-[(hydroxyacetyl)amino]-4-pyridinyl]methyl]thio]-N-[4-(trifluoromethoxy)phenyl]-3-pyridinecarboxamide, or sirolimus.

According to an eleventh aspect of the present invention, the depot formulation as described in any one of the fourth to tenth aspects contains 0.001 to 30% (w/w) of the drug.

According to a twelfth aspect of the present invention, the depot formulation as described in any one of the first to eleventh aspects contains at least 0.1% (w/w) of the trialkyl citrate and/or acetyl trialkyl citrate.

According to a thirteenth aspect of the present invention, the depot formulation as described in any one of the first to twelfth aspects does not contain polylactic acid (PLA) and polylactic acid-glycolic acid copolymer (PLGA).

According to a fourteenth aspect of the present invention, the depot formulation as described in any one of the first to thirteenth aspects does not contain tocopherol.

According to a fifteenth aspect of the present invention, the depot formulation as described in any one of the first to fourteenth aspects is for the prevention and/or treatment of eye disease.

According to a sixteenth aspect of the present invention, the depot formulation as described in any one of the first to fifteenth aspects is for use as a preventive medicine and/or therapeutic medicine of eye disease.

According to a seventeenth aspect of the present invention, in the depot formulation as described in the fifteenth or sixteenth aspect, the eye disease is age-related macular degeneration, retinopathia diabetica, prematurity retinopathy, occlusion of retinal vein, occlusion of retinal artery, polypoidal choroidal vasculopathy, retinal angiomatous proliferation, myopic choroidal neovascularization, diabetic macular edema, eye tumor, radiation retinopathy, rubeosis iridis, rubeotic glaucoma, proliferative vitreoretinopathy (PVR), primary open-angle glaucoma, secondary open-angle glaucoma, normal tension glaucoma, hypersectretion glaucoma, primary angle-closure glaucoma, secondary angle-closure glaucoma, plateau iris glaucoma, combined mechanism glaucoma, developmental glaucoma, steroid induced glaucoma, exfoliation glaucoma, amyloidotic glaucoma, rubeotic glaucoma, malignant glaucoma, glaucoma capsulare of crystalline lens, plateau iris syndrome, hypertonia oculi, uveitis, intraocular infection, or the like.

According to an eighteenth aspect of the present invention, the depot formulation as described in any one of the first to seventeenth aspects is for ocular topical administration.

According to a nineteenth aspect of the present invention, the depot formulation as described in the eighteenth aspect is for intravitreal administration, subconjunctival administration or intracameral administration.

According to a twentieth aspect of the present invention, the depot formulation as described in any one of the first to nineteenth aspects is administered in 1 to 5000 µL one time.

According to a twenty-first aspect of the present invention, the depot formulation as described in any one of the first to twentieth aspects is administered at an interval of one time per three days to one time per five years.

According to a twenty-second aspect of the present invention, the depot formulation as described in any one of the first to twenty-first aspects is for drug sustained release.

A twenty-third aspect of the present invention is the use of a trialkyl citrate and/or acetyl trialkyl citrate for preparation of a depot formulation as described in any one of the first to twenty-second aspects for prevention and/or treatment of eye disease.

According to a twenty-fourth aspect of the present invention is the use of a trialkyl citrate and/or acetyl trialkyl citrate for forming a depot formulation as described in any one of the first to twenty-second aspects.

A twenty-fifth aspect of the present invention is trialkyl citrate and/or acetyl trialkyl citrate for use as a preventive medicine and/or therapeutic medicine of eye disease.

A twenty-sixth aspect of the present invention is the use of a depot formulation as described in any one of the first to twenty-second aspects for the prevention and/or treatment of eye disease.

A twenty-seventh aspect of the present invention is a method of preventing and/or treating eye disease by administering a depot formulation as described in any one of the first to twenty-second aspects to a patient requiring prevention and/or treatment of eye disease.

According to a twenty-eighth aspect of the present invention, a method for forming a depot includes bringing a liquid composition containing a trialkyl citrate and/or an acetyl trialkyl citrate into contact with water, a phosphate buffer solution, body fluid or simulated body fluid,
in which alkyl groups possessed by each of the trialkyl citrate and the acetyl trialkyl citrate are the same or different, and have a number of carbon atoms of 3 to 5.

It should be noted that the respective constitutions of the first to twenty-second aspects can be combined by selecting two or more thereof arbitrarily, and can be applied also to the respective constitutions of the twenty-third to twenty-eighth aspects.

The depot formulation of the present invention is a preparation for which depot formation is easy, is capable of maintaining the depot state for a long period as desired, and in the case of containing a drug, can sustained-release the drug after administered into the body, and has sufficient safety as a pharmaceutical preparation.

### BRIEF DESCRIPTION OF THE DRAWINGS

FIG. 1 provides photographs showing the extent of each depot formation for Comparative Example 1 and Example 1; and
FIG. 2 provides showing pathological evaluation results for a preparation of Comparative Example 9;
FIG. 3 provides photographs showing pathological evaluation results for a depot formulation of an example of the present invention; and
FIG. 4 provides a photograph showing pathological evaluation results for the depot formulation of an example of the present invention.

### PREFERRED MODE FOR CARRYING OUT THE INVENTION

Hereinafter, the present invention will be explained in detail.

The present invention is a depot formulation containing trialkyl citrate and/or acetyl alkyl citrate. The depot formulation of the present invention is a formulation for continuously releasing a drug, and forms a depot (cluster) after being administered inside the body or the like. The state of the depot formulation is not particularly limited, and may be a dissolved state or suspended state. The trialkyl citrate and the acetyl trialkyl citrate, for example, can be each obtained by condensation reaction between citric acid or acetyl citrate, and a compound such as an alcohol providing an alkyl group having a carbon number of 3 to 5. This alkyl group, for which three come to be included in each molecule of this trialkyl citrate and this acetyl trialkyl citrate, may be the same or may be different. This alkyl group is an alkyl group represented by R^{a}, R^{b} and R^{c} in formula (2) described later, and R^{a}, R^{b} and R^{c} in formula (3), and does not include the methyl group constituting the acetyl group in the acetyl trialkyl citrate. The depot formulation of the present invention contains a trialkyl citrate and/or acetyl trialkyl citrate having alkyl groups each with a carbon number of 3 to 5; therefore, depot formation is easy, it is possible to maintain the depot form for a long period as desired, and further, it can also form a depot at a desired site due to the specific gravity being appropriate. The depot formulation of the present invention excels in depot formability in this way, and in the case of containing a drug, also excels in the drug sustained releasability from the depot, and sustained release for a long period as desired is also possible. In addition, the depot formulation of the present invention contains the above-mentioned trialkyl citrate and/or acetyl trialkyl citrate; therefore, it also possesses sufficient safety as a pharmaceutical preparation.

The trialkyl citrate contained in the depot formulation of the present invention is a compound represented by formula (2) below. In formula (2), R^{a}, R^{b} and R^{c} denote the same or different alkyl groups, each having 3 to 5 carbon atoms. This alkyl group is preferably a linear or branched alkyl group, and is more preferably a linear or branched alkyl group having a carbon number of 4. As specific examples, an n-propyl group, n-butyl group, n-pentyl group, isopropyl group, isobutyl group, sec-butyl group, tert-butyl group, isopentyl group, etc. can be given, and the n-butyl group is the most preferable.

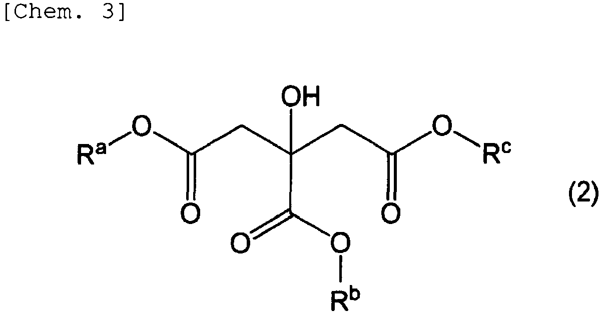

The acetyl trialkyl citrate contained in the depot formulation of the present invention is a compound represented by formula (3) below, and is also referred to as trialkyl acetyl citrate and 2-acetoxypropane-1,2,3-trialkyl tricarboxylic acid. In formula (3), R^{a}, R^{b} and R^{c} each denote alkyl groups having a carbon number of 3 to 5. As this alkyl group, a linear or branched alkyl group is preferably, and a linear or branched alkyl group having a carbon number of 4 is more preferable. As specific examples, an n-propyl group, n-butyl group, n-pentyl group, isopropyl group, isobutyl group, sec-butyl group, tert-butyl group, isopentyl group, etc. can be given, and the n-butyl group is the most preferable.

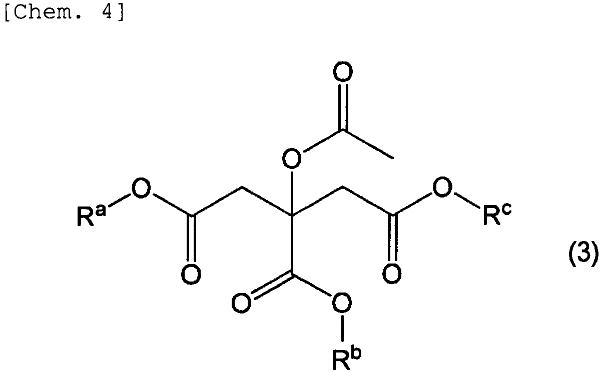

As the above-mentioned trialkyl citrate and/or acetyl trialkyl citrate, only either one of the above-mentioned trialkyl citrate or the above-mentioned acetyl trialkyl citrate may be used, or the above-mentioned trialkyl citrate and the above-mentioned acetyl trialkyl citrate may be used jointly. In the case of using jointly, the content ratio of the above-mentioned trialkyl citrate and the above-mentioned acetyl trialkyl citrate represented by "trialkyl citrate/acetyl trialkyl citrate" is not particularly limited and, for example, may be 0.1/99.9 to 99.9/0.1 by volume ratio, preferably 5/95 to 50/50, more preferably 10/90 to 30/70, and even more preferably 15/85 to 25/75.

As the above-mentioned trialkyl citrate, R^{a}, R^{b} and R^{c} in formula (2) may each be the same, or may be different; however, it is preferable to be the same. As the above-mentioned acetyl trialkyl citrate, R^{a}, R^{b} and R^{c} in formula (3) may each be the same, or may be different; however, it is preferable to be the same.

In the depot formulation of the present invention, the content of the above-mentioned trialkyl citrate and/or acetyl trialkyl citrate is preferably at least 0.1% (w/w), more preferably 0.1 to 99.999% (w/w), even more preferably 1 to 90% (w/w), particularly preferably 2 to 80% (w/w), and most preferably 3 to 70% (w/w). In the case of not blending additives other than a drug in the depot formulation of the present invention, the content of the above-mentioned trialkyl citrate and/or acetyl trialkyl citrate is preferably 70 to 99.999% (w/w), more preferably 75 to 99.99% (w/w), even more preferably 80 to 99.9% (w/w), particularly preferably 85 to 99.5% (w/w), and most preferably 88 to 99% (w/w).

It should be noted that "% (w/w)" denotes mass (g) of target component (herein, trialkyl citrate and/or acetyl trialkyl citrate) contained in 100 g of the depot formulation of the present invention. Hereinafter, it is the same unless otherwise noted.

The depot formulation of the present invention may further contain a drug so long as containing the above-mentioned trialkyl citrate and/or acetyl trialkyl citrate. In the depot formulation of the present invention, there are no particular limitations in the contained drug, and as specific examples, tyrosine kinase inhibitory agents such as Tafetinib, SIM-817378, ACTB-1003, Chiauranib, CT-53608, Cinnamon, chim4G8-SDIE, CEP-5214, IMC-1C11, CEP-7055, 3-[5-[2-[N-(2-methoxyethyl)-N-methylamino]ethoxy]-1H-indol-2-yl]quinolin-2(1H)-one, hF4-3C5, ZK-CDK, IMC-EB10, LS-104, CYC-116, OSI-930, PF-337210, JNJ-26483327, SSR-106462, R-1530, PRS-050, TG-02, SC-71710, SB-1578, AMG-191, AMG-820, Sulfatinib, Lucitanib hydrochloride, JNJ-28312141, Ilorasertib, PLX-5622, ARRY-382, TAS-115, Tanibirumab, Henatinib, LY-2457546, PLX-7486, FPA-008, NVP-AEE-788, cgi-1842, RAF-265, MK-2461, SG-00529, Rebastinib, Golvatinib, Roniciclib, BVT-II, X-82, XV-615, KD-020, Lestaurtinib, Delphinidin, Semaxanib, Vatalanib, OSI-632, Telatinib, Alacizumab pegol, ATN-224, Tivozanib, XL-999, Icrucumab, Foretinib, Crenolanib besylate, R-406, Brivanib, Pegdinetanib, TG-100572, Olaratumab, Fostamatinib disodium, BMS-690514, AT-9283, MGCD-265, Quizartinib, ENMD-981693, Famitinib, Anlotinib, Tovetumab, PLX-3397, Fruquintinib, (-)-Epigallocatechin, Midostaurin, NSC-706456, Orantinib, Cediranib, Dovitinib, XL-647, Motesanib, Linifanib, Brivanib, Cediranib, Apatinib, Fedratinib, Pacritinib, Ramucirumab, Intedanib, Masitinib, Elemene, Dihydroartemisinin, WS-1442, Itraconazole, Leflunomide, Dihydroartemisinin, Imatinib, Sorafenib, Sunitinib, Dasatinib, Pazopanib, Vandetanib, Axitinib, Regorafenib, Cabozantinib, INCB28050 and Ponatinib; steroids such as hydrocortisone, triamcinolone, fluocinolone, dexamethasone, betamethasone and budesonide; prostaglandin derivatives such as isopropyl unoprostone, latanoprost, bimatoprost and travoprost; immunosuppressants such as cyclosporine, sirolimus and FK506; antiallergic agents such as azelastine; non-steroidal anti-inflammatory agents such as indomethacin, bromfenac, diclofenac and nepafenac; antibacterial agents such as Levofloxacin, ofloxacin and gatifloxacin; antihistamines such as olopatadine; angiogenic inhibitors such as pazopanib, SU5416, lapatinib, ranibizumab and bevacizumab; circulation improving agents such as nicardipine and nitrendipine; antioxidants such as Vitamin E; carbonic anhydrase inhibitors such as acetazolamide and brinzolamide; β-receptor antagonists such as timolol and carteolol; visual cycle modulators such as Vitamin A derivatives; nutritional factors and nerve growth factors (NGF) such as cilary growth factor (CNTF) and brain-derived neurotrophic factor (BDNF); growth factors such as stem cell growth factor (HGF); antibody and peptide formulations such as aptamers like pegaptanib, various antisense nucleic acids, nucleic acid medicines like siRNA, Lucentis and IgG; VEGF inhibitors described in Japanese Unexamined Patent Application, Publication No. 2006-96739, Japanese Unexamined Patent Application, Publication No. 2011-37844, Japanese Unexamined Patent Application, Publication No. 2005-232149, Japanese Unexamined Patent Application, Publication No. 2006-273851, Japanese Unexamined Patent Application, Publication No. 2006-306861, Japanese Unexamined Patent Application, Publication No. 2008-266294, etc.; compounds having glucocorticoid receptor avidity described in Japanese Unexamined Patent Application, Publication No. 2007-230993, Japanese Unexamined Patent Application, Publication No. 2008-074829, Japanese Unexamined Patent Application, Publication No. 2008-143889, Japanese Unexamined Patent Application, Publication No. 2008-143890, Japanese Unexamined Patent Application No. 2008-143891, Japanese Unexamined Patent Application, Publication No. 2009-007344, Japanese Unexamined Patent Application, Publication No. 2009-084274, etc.; selective glucocorticoid receptor agonists such as RU24858; anticancer drugs such as fluorouracil; Janus kinase inhibitors such as Tofacitinib; protein kinase inhibitors such as ruboxistaurin mesylate; etc. can be exemplified.

In the depot formulation of the present invention, specific examples of preferred drugs to be contained are compounds represented by formula (1) above and salts thereof.

"Halogen atom" denotes fluorine, chlorine, bromine or iodine.

"C₁₋₆ alkyl group" denotes a linear or branched alkyl group with 1 to 6 carbon atoms, and a linear or branched alkyl group having a carbon number of 1 to 4 is preferable. As specific examples, a methyl group, ethyl group, n-propyl group, n-butyl group, n-pentyl group, n-hexyl group, isopropyl group, isobutyl group, sec-butyl group, tert-butyl group, isopentyl group, etc. can be exemplified.

"C₁₋₆ alkoxy group" denotes a group in which a hydrogen atom of the hydroxyl group has been substituted by the above-mentioned C₁₋₆ alkyl group. As specific examples, a methoxy group, ethoxy group, n-propoxy group, n-butoxy group, n-pentoxy group, n-hexyloxy group, isopropoxy group, isobutoxy group, sec-butoxy group, tert-butoxy group, isopentyloxy group, etc. can be exemplified.

"C₁₋₆ alkyl carbonyl group" denotes a group in which a hydrogen atom of a formyl group has been substituted by the above-mentioned C₁₋₆ alkyl group. As specific examples, a methylcarbonyl group (acetyl group), ethylcarbonyl group, n-propylcarbonyl group, n-butylcarbonyl group, n-pentylcarbonyl group, n-hexylcarbonyl group, isopropylcarbonyl group, isobutylcarbonyl group, sec-butylcarbonyl group, tert-butylcarbonyl group, isopentylcarbonyl group, etc. can be exemplified.

"Substituted by one or a plurality of halogen atoms" referred to in the present invention denotes the matter of the above-mentioned C₁₋₆ alkyl group (including the above-mentioned C₁₋₆ alkyl group constituting the above-mentioned C₁₋₆ alkoxy group) being substituted by at least one, i.e. a number no more than the possible number of substitutions, of halogen atoms. Each of the halogen atoms may be the same or different, a case of the number of halogen atoms being 2 or 3 is preferable, and the case of being 3 is particularly preferable.

"Substituted by one or a plurality of hydroxyl groups" referred to in the present invention denotes the matter of the above-mentioned C₁₋₆ alkyl group being substituted by at least one, i.e. a number no more than the possible number of substitutions, of halogen atoms. For the number of hydroxyl groups, a case of being 1 or 2 is preferable, and the case of being 1 is particularly preferable.

In addition, the drug in the present invention also encompasses derivatives such as esters, amides and acetonides of compounds having pharmacological activity. These derivatives may be prodrugs of these compounds having pharmacological activity. As specific examples of esters, esters produced by a carboxylic acid such as acetic acid, propionic acid, isopropionic acid, butyric acid, isobutyric acid and pivalic acid condensing with a hydroxyl group in the drug can be exemplified. As specific examples of amides, amides produced by a carboxylic acid such as acetic acid, propionic acid, isopropionic acid, butyric acid, isobutyric acid and pivalic acid condensing with an amino group in the drug can be exemplified. As specific examples of acetonides, acetonides (acetals) produced by two hydroxyl groups in the drug (1,2-diol or 1,3-diol) reacting with acetone or an equivalent thereof (2,2dimethoxypropane, etc.) can be exemplified.

In addition, the contained drug may assume the form of a hydrate or a solvate.

In the case of a geometric isomer, tautomer or enantiomer existing in the contained drug, isomers thereof are also included in the scope of the present invention.

Furthermore, in the case of crystalline polymorphism existing for the contained drug, the crystalline polymorph is also included in the scope of the present invention.
(a) As a preferred example of the compound represented by formula (1), compounds in which the respective groups in formula (1) are the groups shown below or salts thereof can be exemplified.
(a1) R¹ denotes a C₁₋₆ alkoxy group or a C₁₋₆ alkoxy group substituted by one or a plurality of halogen atoms; and/or
(a2) R² denotes a C₁₋₆ alkyl carbonyl group or a C₁₋₆ alkyl carbonyl group substituted by one or a plurality of hydroxyl groups.

In other words, in the compound represented by formula (1), compounds consisting of one or the respective combinations of two or more selected from the above (a1) and (a2) or salts thereof can be exemplified as preferred examples.
(b) As more preferred examples of compounds represented by formula (1), compounds of formula (1) in which each group is a group shown below or salts thereof can be exemplified.
(b1) R¹ denotes a C₁₋₆ alkoxy group substituted by one or a plurality of halogen atoms; and/or
(b2) R² denotes a C₁₋₆ alkyl carbonyl group substituted by one or a plurality of hydroxyl groups.

In other words, of the compounds represented by formula (1), compounds consisting of one, or the respective combinations of two or more, selected from the above (b1) and (b2) or salts thereof can be exemplified as preferred examples. In addition, the selected conditions thereof can also be combined with the conditions of (a).
(c) As most preferred examples of compounds represented by formula (1), compounds represented by formula (4): 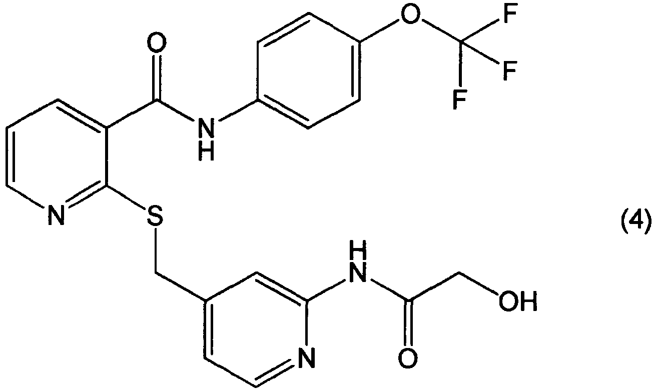 (2-[[[2-[(hydroxyacetyl)amino]-4-pyridinyl]methyl]thio]-N-[4-(trifluoromethoxy)phenyl]-3-pyridinecarboxamide) or salts thereof can be exemplified.

The compounds represented by formula (1), or salts thereof, contained in the depot formulation of the present invention can be prepared following a normal method in this technical field, such as the method described in the specification of US Patent Application, Publication No. 2007/0149574.

In the depot formulation of the present invention, other preferred specific examples of contained drugs are 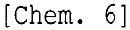 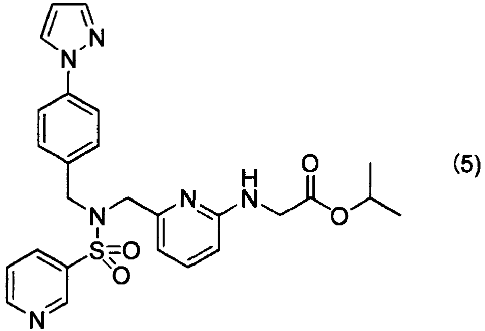 isopropyl (6-{[4-(pyrazol-1-yl)benzyl](pyridin-3-ylsulfonyl)aminomethyl}pyridin-2-ylamino)acetate represented by formula (5) or a salt thereof.

The compound represented by formula (5), or salt thereof, contained in the depot formulation of the present invention can be prepared following a normal method in the technical field, such as the methods described in the specification of US Patent Application, Publication No. 2011/0054172 and the specification of US Patent Application, Publication No. 2012/0190852.

In the depot formulation of the present invention, the contained drug may be a salt, and is not particularly limited so long as being a pharmaceutically allowable salt. As the salt, a salt with an inorganic acid, a salt with an organic acid, a quaternary ammonium salt, a salt with a halogen ion, a salt with an alkali metal, a salt with an alkali earth metal, a metal salt, a salt with organic amine, etc. can be exemplified. As salts of inorganic acids, salts of hydrochloric acid, hydrobromic acid, hydroiodic acid, nitric acid, sulfuric acid, phosphoric acid, etc. can be exemplified. As salts of organic acids, salts of acetic acid, oxalic acid, fumaric acid, maleic acid, succinic acid, malic acid, citric acid, tartaric acid, adipic acid, gluconic acid, glucoheptonic acid, glucuronic acid, terephthalic acid, methanesulfonic acid, alanine, lactic acid, hippuric acid, 1,2-ethanedisulfonic acid, isethionic acid, lactobionic acid, oleic acid, gallic acid, pamoic acid, polygalacturonic acid, stearic acid, tannic acid, trifluoromethanesulfonic acid, benzene-sulfonic acid, p-toluenesulfonic acid, lauryl sulfate, methyl sulfate, naphthalenesulfonic acid, sulfosalicyclic acid, etc. can be exemplified. As the tertiary ammonium salt, salts with methyl bromide, methyl iodide, etc. can be exemplified. As salts with a halogen ion, salts with a chloride ion, bromide ion, iodide ion, etc. can be exemplified; as salts with an alkyl metal, salts with lithium, sodium, potassium, etc. can be exemplified; as salts with an alkali earth metal, salts with calcium, magnesium, etc. can be exemplified; and as metal salts, salts with silver, zinc, etc. can be exemplified. As salts with an organic amine, salts with triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, N,N-bis(phenylmethyl)-1,2-ethanediamine, etc. can be exemplified.

In the depot formulation of the present invention, the content of the contained drug is not particularly limited so long as being a sufficient amount to exert the desired drug efficacy, and 0.001 to 30% (w/v) is preferable, 0.01 to 25% (w/v) is more preferable, 0.1 to 20% (w/v) is even more preferable, 0.5 to 15% (w/v) is even further preferably, 1 to 12% (w/v) is particularly preferable, and 1% (w/v), 1.5% (w/v), 2% (w/v), 2.5% (w/v), 3% (w/v), 3.5% (w/v), 4% (w/v), 5% (w/v), 6% (w/v), 7% (w/v), 8% (w/v), 9% (w/v), 10% (w/v), 11% (w/v) or 12% (w/v) is most preferable. It should be noted that "% (w/v)" denotes the mass (g) of target component (herein drug) contained in 100 mL of the depot formulation of the present invention. Hereinafter, it is the same unless otherwise noted.

Additives can be used in the depot formulation of the present invention as necessary, and as the additives, surfactants, buffering agents, isotonizing agents, stabilizers, antiseptics, antioxidants, high molecular weight polymers, diluting agents, solvents, etc. can be added.

In the depot formulation of the present invention, surfactants, e.g., cationic surfactants, anionic surfactants and non-ionic surfactants, which are usable as additives in pharmaceutical preparations, can be blended. As examples of the anionic surfactant, phospholipids, etc. can be exemplified, and lecithin, etc. can be exemplified as phospholipids. As examples of the cationic surfactant, alkyl amine salts, polyoxyethylene adducts of alkylamines, fatty acid triethanolamine monoester salts, acylamino ethyldiethylamine salts, fatty acid polyamine condensates, alkyltrimethyl ammonium salts, dialkyl dimethyl ammonium salts, alkyl dimethyl benzyl ammonium salts, alkylpyridinium salts, acylamino alkyl-type ammonium salts, acylamino alkylpyridinium salts, diacyloxyethyl ammonium salts, alkyl imidazoline, 1-acylaminoethyl-2-alkylimidazoline, 1-hydroxyethyl-2-alkylimidazoline, etc. can be exemplified. As the alkyldimethylbenzyl ammonium salt, benzalkonium chloride, cetalkonium chloride, etc. can be exemplified. As examples of the non-ionic surfactant, polyoxyethylene fatty acid esters, polyoxyethylene sorbitan fatty acid esters, polyoxyethylene hydrogenated castor oil, polyoxyethylene castor oil, polyoxyethylene polyoxypropylene glycol, sucrose fatty acid ester, Vitamin E TPGS, etc. can be exemplified.

As the polyoxyethylene fatty acid ester, polyoxyl stearate 40, etc. can be exemplified.

As the polyoxyethylene sorbitan fatty acid ester, polysorbate 80, polysorbate 60, polysorbate 40, polysorbate 20, polyoxyethylene sorbitan monolaurate, polyoxyethylene sorbitan triolate, polysorbate 65, etc. can be exemplified.

As the polyoxyethylene hydrogenated castor oil, it is possible to various polyoxyethylene hydrogenated castor oils having different polymerization numbers of ethylene oxide, and the polymerization number of ethylene oxide is preferably 10 to 100, more preferably 20 to 80, particularly preferably 40 to 70, and most preferably 60. As specific examples of polyoxyethylene hydrogenated castor oils, polyoxyethylene hydrogenated castor oil 10, polyoxyethylene hydrogenated castor oil 40, polyoxyethylene hydrogenated castor oil 50, polyoxyethylene hydrogenated castor oil 60, etc. can be exemplified.

As the polyoxyethylene castor oil, it is possible to use various polyoxyethylene castor oils having different polymerization numbers of ethylene oxide, and the polymerization number of ethylene oxide is preferably 5 to 100, more preferably 20 to 50, particularly preferably 30 to 40, and most preferably 35. As specific examples of polyoxyethylene castor oils, polyoxyl 5 castor oil, polyoxyl 9 castor oil, polyoxyl 15 castor oil, polyoxyl 35 castor oil, polyoxyl 40 castor oil, etc. can be exemplified.

As the polyoxyethylene polyoxypropylene glycol, polyoxyethylene (160) polyoxypropylene (30) glycol, polyoxyethylene (42) polyoxypropylene (67) glycol, polyoxyethylene (54) polyoxypropylene (39) glycol, polyoxyethylene (196) polyoxypropylene (67) glycol, polyoxyethylene (20) polyoxypropylene (20) glycol, etc. can be exemplified.

As the sucrose fatty acid ester, sucrose stearate, etc. can be exemplified.

Vitamin E TPGS is also referred to as tocopherol polyethylene glycol 1000 succinate.

Buffer agents that can be used as additives of pharmaceutical preparations can be blended into the depot formulation of the present invention. As the buffer agent, phosphoric acid or a salt thereof, boric acid or a salt thereof, citric acid or a salt thereof, acetic acid or a salt thereof, carbonic acid or a salt thereof, tartaric acid or a salt thereof, histidine or a salt thereof, ε-aminocaprionic acid, trometamol, etc. can be exemplified. As the salt of phosphoric acid, sodium phosphate, sodium dihydrogenphosphate, disodium hydrogenphosphate, potassium phosphate, potassium dihydrogenphosphate, dipotassium hydrogenphosphate, etc. can be exemplified; as the salt of boric acid, borax, sodium borate, potassium borate, etc. can be exemplified; as the salt of citric acid, sodium citrate, disodium citrate, etc. can be exemplified; as the salt of acetic acid, sodium acetate, potassium acetate, etc. can be exemplified; as the salt of carbonic acid, sodium carbonate, sodium hydrogencarbonate, etc. can be exemplified; as the salt of tartaric acid, sodium tartrate, potassium tartrate, etc. can be exemplified; and as the histidine salt, histidine hydrochloride, etc. can be exemplified.

Isotonizing agents that can be used as additives of pharmaceutical preparations can be blended into the depot formulation of the present invention as appropriate. As examples of the isotonizing agent, ionic isotonizing agents, non-ionic isotonizing agents, etc. can be exemplified. As the ionic isotonizing agent, sodium chloride, potassium chloride, calcium chloride, magnesium chloride, etc. can be exemplified; and as the non-ionic isotonizing agent, glycerine, propylene glycol, sorbitol, mannitol, trehalose, sucrose, glucose, etc. can be exemplified.

Stabilizers that can be used as additives of pharmaceutical preparations can be blended into the depot formulation of the present invention as appropriate. As examples of the stabilizer, edetic acid, disodium edetate, sodium citrate, etc. can be exemplified.

Antiseptics that can be used as additives of pharmaceutical preparations can be blended into the depot formulation of the present invention as appropriate. As examples of the antiseptic, benzalkonium chloride, benzalkonium bromide, benzethonium chloride, sorbic acid, potassium sorbate, methyl parahydroxybenzoate, propyl parahydroxybenzoate, chlorobutanol, etc. can be exemplified.

Antioxidants that can be used as additives of pharmaceutical preparations can be blended into the depot formulation of the present invention as appropriate. As examples of the antioxidant, ascorbic acid, tocopherol, dibutyl hydroxytoluene, butylated hydroxyanisole, sodium erythoribate, propyl gallate, sodium sulfite, or derivatives thereof, etc. can be exemplified, and tocopherol or derivatives thereof are preferable. As tocopherol or derivatives thereof, Vitamin E, α-tocopherol, β-tocopherol, γ-tocopherol, δ-tocopherol, and acetate esters, succinic acid esters thereof, as well as d form, 1 form, dl forms thereof, etc. can be exemplified.

High molecular weight polymers that can be used as additives of pharmaceutical preparations can be blended into the depot formulation of the present invention as appropriate. As examples of the high molecular weight polymer, methyl cellulose, ethyl cellulose, hydroxymethyl cellulose, hydroxyethyl cellulose, hydroxypropyl cellulose, hydroxyethylmethyl cellulose, hydroxypropylmethyl cellulose, carboxymethyl cellulose, sodium carboxymethyl cellulose, hydroxypropyl methylcellulose acetate succinate, hydroxypropyl-methylcellulose phthalate, carboxymethylethyl cellulose, cellulose acetate phthalate, polyvinyl pyrrolidone, polyvinyl alcohol, carboxyvinyl polymer, etc. can be exemplified.

The depot formulation of the present invention may be one containing at least one additive selected from the group consisting of polylactic acid (PLA) and polylactic acid-glycolic acid copolymer (PLGA). On the other hand, by containing the aforementioned trialkyl citrate and/or acetyl trialkyl citrate, since it is possible to form a sustained-release depot easily, it is unnecessary to contain at least one additive selected from the group consisting of PLA and PLGA. The depot formulation of the present invention preferably does not contain these additives in the point of being able to perform appropriate viscosity adjustment easily by not containing at least one additive selected from the group consisting of tocopherol acetate, PLA and PLGA. Conventionally, depot formulations containing these additives tend to have higher viscosity and the operability may deteriorate depending on the concentration of these additives. In addition, in the case of containing a drug in particular, the depot formulation of the present invention preferably does not contain tocopherol acetate in the point of appropriate disappearance after administration.

The content of additives in the case of blending additives into the depot formulation of the present invention can be appropriately adjusted according to the type, etc. of additive; however, as the total amount thereof, 0.0001 to 30% (w/v) is preferable, 0.001 to 25% (w/v) is more preferable, 0.01 to 20% (w/v) is even more preferable, 0.1 to 15% (w/v) is particularly preferable, and 1 to 10% (w/v) is the most preferable.

Solvents or excipients that can be used as additives of pharmaceutical preparations can be blended into the depot formulation of the present invention as appropriate. As examples of the solvent or excipient, polyethylene glycol (PEG), glycofurol, dimethylsulfoxide, N-methylpyrrolidone, N,N-dimethylacetamide, ethanol, benzyl benzoate, sucrose octaacetate, medium chain fatty acid triglycerides, vegetable oils such as castor oil, mineral oils such as liquid paraffin, silicone oil, etc. can be exemplified, and polyethylene glycol and benzyl benzoate are preferable. As the solvent or excipient, only one may be used, or two or more may be used jointly. For example, in the case of jointly using polyethylene glycol and benzyl benzoate, the content ratio of polyethylene glycol to benzyl benzoate represented by "polyethylene glycol/benzyl benzoate" is not particularly limited and, for example, by volume ratio, may be 0.1/99.9 to 99.9/0.1, preferably 5/95 to 70/30, and more preferably 10/90 to 50/50.

In the case of blending polyethylene glycol into the depot formulation of the present invention, the average molecular weight thereof is preferably 100 to 2000, more preferably 150 to 1500, even more preferably 200 to 1300, particularly preferably 300 to 1200, and most preferably 400 to 1000. As specific examples of the polyethylene glycol, PEG 100, PEG 200, PEG 300, PEG 400, PEG 600, PEG 800, PEG 1000, etc. can be exemplified.

The content of solvent or excipient in the case of blending a solvent or excipient into the depot formulation of the present invention is preferably 5 to 99% (w/w), more preferably 10 to 98% (w/w), even more preferably 30 to 97% (w/w), and most preferably 40 to 95% (w/w).

The depot formulation of the present invention, if containing a trialkyl citrate and/or acetyl trialkyl citrate having alkyl groups with a carbon number of 3 to 5 as mentioned above, it may further contain at least one citric acid derivative selected from the group consisting of a trialkyl citrate in which at least one of R^{a}, R^{b} and R^{c} in the aforementioned formula (2) is an alkyl group with a carbon number of at least 6 (e.g., trihexyl citrate), and an acetyl trialkyl citrate in which at least one among R^{a}, R^{b} and R^{c} in formula (3) is an alkyl group with a carbon number of at least 6 (e.g., acetyl trihexyl citrate). The content in the case of blending these additional citric acid derivatives into the depot formulation of the present invention is preferably 5 to 99% (w/w), more preferably 10 to 98% (w/w), even more preferably 30 to 97% (w/w), and most preferably 40 to 95% (w/w).

For the depot formulation of the present invention, a specific embodiment is a depot formulation substantially only containing a compound represented by formula (4) or a salt thereof, and tri-n-butyl citrate.

For the depot formulation of the present invention, another specific embodiment is a depot formulation substantially only containing a compound represented by formula (4) or a salt thereof, tri-n-butyl citrate and PEG 400.

For the depot formulation of the present invention, another specific embodiment is a depot formulation substantially only containing a compound represented by formula (4) or a salt thereof, and acetyl tri-n-butyl citrate.

For the depot formulation of the present invention, another specific embodiment is a depot formulation substantially only containing a compound represented by formula (4) or a salt thereof, acetyl tri-n-butyl citrate and PEG 400.

For the depot formulation of the present invention, another specific embodiment is a depot formulation substantially only containing a compound represented by formula (4) or a salt thereof, tri-n-butyl citrate, acetyl tri-n-butyl citrate and PEG 400.

For the depot formulation of the present invention, a specific embodiment is a depot formulation substantially only containing a compound represented by formula (5) or a salt thereof, and tri-n-butyl citrate.

For the depot formulation of the present invention, another specific embodiment is a depot formulation substantially only containing a compound represented by formula (5) or a salt thereof, tri-n-butyl citrate and PEG 400.

For the depot formulation of the present invention, another specific embodiment is a depot formulation substantially only containing a compound represented by formula (5) or a salt thereof, and acetyl tri-n-butyl citrate.

For the depot formulation of the present invention, another specific embodiment is a depot formulation substantially only containing a compound represented by formula (5) or a salt thereof, acetyl tri-n-butyl citrate, and PEG 400.

For the depot formulation of the present invention, another specific embodiment is a depot formulation substantially only containing a compound represented by formula (5) or a salt thereof, acetyl tri-n-butyl citrate, PEG 400 and benzyl benzoate.

The depot formulation of the present invention can be administered either orally or parenterally. The dosage form of the depot formulation of the present application is not particularly limited so long as being usable as a pharmaceutical preparation. As the dosage form, for example, if an oral formulation, a liquid formulation and suspension can be exemplified, and if a parenteral formulation, an injection, transfusion, nasal drops, ear drops, eye drops, etc. can be exemplified. Ophthalmologic injections and eye drops are preferably exemplified, ophthalmologic injections are more preferably exemplified, and intravitrally, intracamerally administered or subconjunctivally administered injections are most preferably exemplified. These can be prepared following a common method in the present technical field.

The depot formulation of the present invention can be administered as appropriate according to the dosage form thereof. For example, in the case of an ophthalmologic injection, it is possible to administer intravitreally, posterior juxtasclerally, periorbitally, and between sclera and conjunctiva. For example, in the case of administering an ophthalmologic injection intravitreally, there is no particular limitation in dosage so long as being a sufficient amount to exert the desired drug efficacy; however, for one time, 1 to 5000 µL is preferable, 5 to 1000 µL is more preferable, 10 to 100 µL is even more preferable, 20 to 50 µL is particularly preferable, and 20 µL, 30 µL, 40 µL or 50 µL is the most preferable. In the case of administering an ophthalmologic injection intracamerally, there is no particular limitation in dosage so long as being a sufficient amount to exert the desired drug efficacy; however, for one time, 0.1 to 300 µL is preferable, 1 to 100 µL is more preferable, 2 to 50 µL is even more preferable, 5 to 20 µL is particularly preferable, and 5 µL, 10 µL, 15 µL or 20 µL is most preferable. In the case of administering an ophthalmologic injection subconjunctivally, there is no particular limitation in dosage so long as being a sufficient amount to exert the desired drug efficacy; however, for one time, 10 to 5000 µL is preferable, 20 to 1000 µL is more preferable, 30 to 500 µL is even more preferable, 50 to 200 µL is particularly preferable, and 50 µL, 100 µL, 150 µL or 200 µL is most preferable. The dosage of drug is preferably 0.001 to 30 g/eye, more preferably 0.01 to 10 mg/eye, even more preferably 0.1 to 5 mg/eye, particularly preferably 0.2 to 1.6 mg/eye, and most preferably 0.2 mg/eye, 0.3 mg/eye, 0.4 mg/eye, 0.5 mg/eye, 0.6 mg/eye, 0.7 mg/eye, 0.8 mg/eye, 1 mg/eye, 1.2 mg/eye, 1.4 mg/eye or 1.6 mg/eye.

In the case of successively administering the depot formulation of the present invention intravitreally or intracamerally, there is no particular limitation in dosage interval so long as being a sufficient to exert the desired drug efficacy; however, being administered at an interval of once in 3 days to once in 5 years is preferable, being administered at an interval of once in 3 days, once in 5 days, once in 1 week, once in 2 weeks, once in 1 month, once in 2 months, once in 3 months, once in 4 months, once in 5 months, once in 6 months, once in 1 year, once in 2 years, once in 3 years, once in 4 years or once in 5 years is more preferable, and being administered at an interval of once in 2 months, once in 3 months, once in 4 months, once in 5 months, once in 6 months or once in 1 year is most preferable. In addition, the dosage interval can be changed as appropriate.

The depot formulation of the present invention is useful as a medicine, and eye diseases, e.g., age-related macular degeneration, retinopathia diabetica, prematurity retinopathy, occlusion of retinal vein, occlusion of retinal artery, polypoidal choroidal vasculopathy, retinal angiomatous proliferation, myopic choroidal neovascularization, diabetic macular edema, eye tumor, radiation retinopathy, rubeosis iridis, rubeotic glaucoma, proliferative vitreoretinopathy (PVR), primary open-angle glaucoma, secondary open-angle glaucoma, normal tension glaucoma, hypersectretion glaucoma, primary angle-closure glaucoma, secondary angle-closure glaucoma, plateau iris glaucoma, combined mechanism glaucoma, developmental glaucoma, steroid induced glaucoma, exfoliation glaucoma, amyloidotic glaucoma, rubeotic glaucoma, malignant glaucoma, glaucoma capsulare of crystalline lens, plateau iris syndrome, hypertonia oculi, uveitis, intraocular infection, etc. can be exemplified. As the disease, it can more preferably be used as a preventative or therapeutic agent for age-related macular degeneration, diabetic retinopathy, primary open-angle glaucoma, normal tension glaucoma, primary angle-closure glaucoma, ocular hypertension, uveitis, intraocular infection, etc.

The depot formulation, in the case of not containing drug, for example, can be used in the testing of depot formation property of the entire formulation upon formulation design, administration practicing, etc.

The depot formulation of the present invention, also in the case of containing drug, can be used as an injection for the prevention and/or treatment of eye disease. As such a depot formulation, since it is possible to form a depot in the vicinity of the administration site if administered intravitreally, for example, it is possible to effectively and continuously supply the drug to the affect part by the eye disease (e.g., chorioretinal atrophy).

The detailed explanation of the depot formulation of the present invention described above is also applied to trialkyl citric acid and/or acetyl trialkyl citric acid for use as a preventive medicine or therapeutic medicine of eye disease, and a depot formation method of the present invention.

A depot formation method including bringing a liquid composition containing a trialkyl citrate and/or acetyl trialkyl citrate into contact with water, a phosphate buffer solution, body fluid or simulated body fluid, in which the alkyl groups possessed by each of the trialkyl citrate and the acetyl trialkyl citrate are the same or different, and have a number of carbon atoms of 3 to 5 is also one aspect of the present invention. The above-mentioned trialkyl citrate and/or acetyl trialky citrate are the same as the trialkyl citrate and/or acetyl trialkyl citrate which are essential components of the depot formulation of the present invention. As the body fluid, for example, lacrimal fluid, aqueous humor, vitreous humor, etc. can be exemplified.

Although formulation examples and test results are shown below, these are for better understanding the present invention, and are not to limit the scope of the present invention.

### EXAMPLES

### Formulation Examples

Representative formulation examples of the present invention will be shown below.

### Formulation Example 1

Drug 4 g
Tri-n-butyl citrate 45 g
PEG 400 55 g

### Formulation Example 2

Drug 4 g
Tri-n-butyl citrate 60 g
PEG 400 40 g

### Formulation Example 3

Drug 4 g
Acetyl tri-n-butyl citrate 45 g
PEG 400 55 g

### Formulation Example 4

Drug 4 g
Acetyl tri-n-butyl citrate 60 g
PEG 400 40 g

### Formulation Example 5

Drug 4 g
Acetyl tri-n-butyl citrate 25 g
Benzyl benzoate 25 g
PEG 400 50 g

### Formulation Example 6

Drug 4 g
Acetyl tri-n-butyl citrate 30 g
Benzyl benzoate 30 g
PEG 400 40 g

It should be noted that the type of drug, trialkyl citrate, acetyl trialkyl citrate, additives and solvent and blending amounts in the above-mentioned Formulation Examples 1 to 6 can be appropriately adjusted to obtain the desired depot formulation.

### 1. Depot Disappearance Test

Disappearance in solvent of acetyl triethyl citrate (ATEC) and acetyl tri-n-butyl citrate (ATBC) was evaluated.

### 1.1 Preparation of Release Solvent

To a beaker, 800 mL of water for injection, 1 g of polysorbate 80, 3 g of sodium dihydrogen phosphate, and 29 g of dibasic sodium phosphate hydrate were added, then stirred to dissolve. It was diluted in a measuring cylinder to 1 L with water for injection.

### 1.2 Disappearance Test

To a 5-mL standard bottle, 5 mL of release solvent heated to 37°C was added. To this release solvent, 0.05 mL of test article was administered using a Hamilton syringe equipped with a 30 G needle, and was visually observed immediately after administration, and after one day and thirty-seven days from administration.

### 1.3 Test Results and Considerations

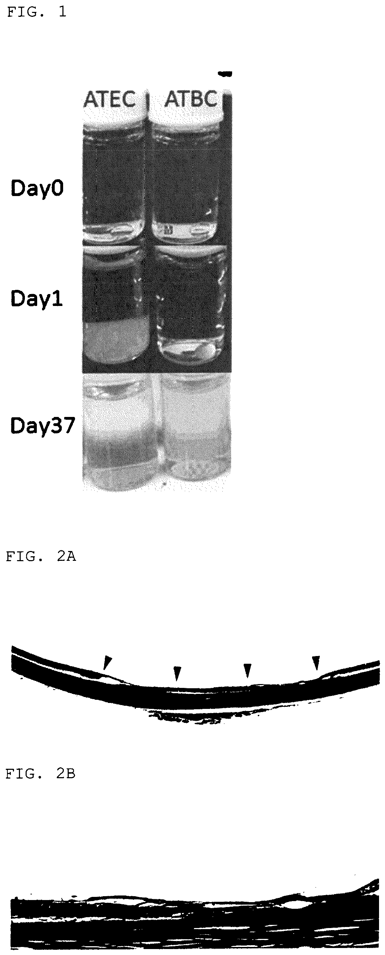

The test results are shown in Table 1. Photographs of the test results are shown in FIG. 1.

**[Table 1]**

| | Comparative Example1 | Example1 |
|---|---|---|
| Test article | ATEC | ATBC |
| Immediately after administration | Transparent, colorless depot | Transparent, colorless depot |
| After 1 day | White, translucent depot and dense, cloudy substance | Colorless, translucent depot |
| After 37 day | Small white, translucent depot | White, translucent depot and dilute, cloudy substance |

As shown in Table 1, for the preparation of Example 1, the formed depot was maintained for a long period compared to the formulation of Comparative Example 1, and the depot was also confirmed thirty-seven days after administration. Based on the above, the depot formulation of the present invention was confirmed as being able to maintain a depot state for a long period after being administered. In addition, it is suggested that drug can be sustained-released over a long period in the case of the depot formulation containing the drug.

### 2. Study of Formulating of Depot Formulation using Each Drug

The formulating of depot formulations with various drugs was studied using acetyl tri-n-butyl citrate (ATBC).

### 2.1 Formulating Study 1

In standard bottles, 30 mg of Nepafenac, Dexamethasone, Indomethacin, Diclofenac Sodium, Levofloxacin, Timolol maleate, Fluocinolone acetonide, Triamcinolone acetonide and Budesonide were respectively weighed, 0.3 mL of dimethyl sulfoxide was added to dissolve, 2.7 mL of acetyl tri-n-butyl citrate or tri-n-butyl citrate was further added and mixed to prepare the formulations of Examples 2 to 8, and Examples 13 to 19.

In standard bottles, 30 mg of INCB28050 was weighed, 1.5 mL of dimethylsulfoxide was added to dissolve, 1.5 mL of acetyl tri-n-butyl citrate or tri-n-butyl citrate was further added and mixed to prepare the formulations of Example 9 and Example 10.

In standard bottles, 30 mg of Ciclosporin A was weighed, 3 mL of acetyl tri-n-butyl citrate or tri-n-butyl citrate was added to dissolve and mixed, thereby preparing the formulations of Examples 11 and 12.

**[Table 2]**

| Formula | Example 2 | Example 3 | Example 4 | Example 5 | Example 6 | Example 7 | Example 8 |
|---|---|---|---|---|---|---|---|
| Nepafenac | 30 mg | 30 mg | - | - | - | - | - |
| Dexamethasone | - | - | 30 mg | 30 mg | - | - | - |
| Indomethacin | - | - | - | - | 30 mg | - | - |
| Diclofenac Sodium | - | - | - | - | - | 30 mg | - |
| Levofloxacin | - | - | - | - | - | - | 30 mg |
| Acetyl tri-n-butyl citrate | 2.7 mL | - | 2.7 mL | - | 2.7 mL | 2.7 mL | 2.7 mL |
| Tri-n-butyl citrate | - | 2.7 mL | - | 2.7 mL | - | - | - |
| Dimethylsulfoxide | 0.3 mL | 0.3 mL | 0.3 mL | 0.3 mL | 0.3 mL | 0.3 mL | 0.3 mL |

**[Table 3]**

| Formula | Example 9 | Example 10 | Example 11 | Example 12 | Example 13 |
|---|---|---|---|---|---|
| INCB28050 | 30 mg | 30 mg | - | - | - |
| Ciclosporin A | - | - | 30 mg | 30 mg | - |
| Timolol maleate | - | - | - | - | 30 mg |
| Acetyl tri-n-butyl citrate | 1.5 mL | - | 3 mL | - | 2.7 mL |
| Tri-n-butyl citrate | - | 1.5 mL | - | 3 mL | - |
| Dimethylsulfoxide | 1.5 mL | 1.5 mL | - | - | 0.3 mL |

**[Table 4]**

| Formula | Example 14 | Example 15 | Example 16 | Example 17 | Example 18 | Example 19 |
|---|---|---|---|---|---|---|
| Fluocinolone acetonide | 30 mg | 30 mg | - | - | - | - |
| Triamcinolone acetonide | - | - | 30 mg | 30 mg | - | - |
| Budesonide | - | - | - | - | 30 mg | 30 mg |
| Acetyl tri-n-butyl citrate | 2.7 mL | - | 2.7 mL | - | 2.7 mL | - |
| Tri-n-butyl citrate | - | 2.7 mL | - | 2.7 mL | - | 2.7 mL |
| Dimethylsulfoxide | 0.3 mL | 0.3 mL | 0.3 mL | 0.3 mL | 0.3 mL | 0. 3 mL |

### 2.2 Formulating Study 2

In standard bottles, 5 mg of olopatadine was weighed, 0.5 g of dimethyl sulfoxide was added to dissolve, and acetyl tri-n-butyl citrate was further added and mixed so as to make the total mass of formulation become 5 mL, thereby preparing the formulation of Example 20.

**[Table 5]**

| Formula | Example 20 |
|---|---|
| Olopatadine | 5 mg |
| Acetyl tri-n-butyl citrate | q.s. |
| Dimethylsulfoxide | 0.5 g |
| Total amount | 5 mL |

Since the respective formulations shown in Tables 2 to 5 dissolve all the drugs, it was suggested that the depot formulation of the present invention is able to be formulated also with various drugs.

### 3. Sustained Releasability Evaluation Test (1)

The drug sustained releasability of the depot formulation of the present invention was evaluated.

### 3.1 Preparation of Test Article

In a standard bottle, 10 mg of latanoprost was measured, and dissolved by adding 1 mL of acetyl triethyl citrate, thereby preparing the formulation of Comparative Example 2. In addition, 50 mg of latanoprost was measured in a standard bottle, and dissolved by adding 5 mL of tri-n-butyl citrate or acetyl tri-n-butyl citrate, thereby preparing the formulations of Examples 21 and 22, respectively.

### 3.2 Preparation of Release Solvent

To a beaker, 800 mL of water for injection, 1 g of polysorbate 80, 3 g of sodium dihydrogen phosphate, and 29 g of dibasic sodium phosphate hydrate were added, then stirred to dissolve. It was diluted in a measuring cylinder to 1 L with water for injection.

### 3.3 Release Test

To a 5-mL standard bottle, 5 mL of release solvent heated to 37°C was added. Using a Hamilton syringe equipped with a 30 G needle, 0.025 mL of the test article was administered, and stirred at 37°C and 86 rpm. One, four, seven, eleven, fourteen, eighteen, twenty-one, twenty-five and twenty-eight days after test article administration, 0.75 mL of release solution was collected, and diluted with 0.75 mL of acetonitrile/water (1:1 by volume ratio). To a standard bottle, 0.75 mL of new release solvent was added. The content of latanoprost in the collected release solvent was measured by HPLC, and the cumulative release rate after administration was calculated.

### 3.4 Test Results and Considerations

The test results are shown in Table 2.

**[Table 6]**

| Formula | | Comparative Example 2 | Example 21 | Example 22 |
|---|---|---|---|---|
| Latanoprost | | 10 mg | 50 mg | 50 mg |
| Acetyl triethyl citrate | | 1 mL | - | - |
| Tri-n-butyl citrate | | - | 5 mL | - |
| Acetyl tri-n-butyl citrate | | - | - | 5 mL |
| Cumulative | After 1 | 16.2 | 7.3 | 17.5 |
| release rate of latanoprost (%) | day | | | |
| | After 4 days | 41.7 | 24.4 | 46.3 |
| | After 7 days | 73.0 | 39.6 | 64.8 |
| | After 11 days | 107.7 | 54.4 | 80.3 |
| | After 14 days | - | 63.4 | 89.1 |
| | After 18 days | - | 74.6 | 97.0 |
| | After 21 days | - | 80.9 | 101.5 |
| | After 25 days | - | 87.3 | 105.1 |
| | After 28 days | - | 91.7 | 107.2 |

As shown in Table 6, 107.7% of latanoprost was released by the formulation of Comparative Example 2 eleven days after administration; whereas, the formulations of Examples 21 and 22 only released 54.4 to 80.3% of latanoprost eleven days after administration. Furthermore, the formulations of Examples 21 and 22 showed sustained release until twenty-eight days after administration. Based on the above, the depot formulation of the present invention was confirmed to sustained-release the drug. In addition, the formulation of Example 21 prepared using tri-n-butyl citrate as the base had higher controlled releasability than the formulation of Example 22 prepared using acetyl tri-n-butyl citrate as the base. Based on this fact, it was confirmed that it is possible to control the sustained releasability by selecting the type of base, for example, according to the intended extent of sustained releasability 4. Sustained Releasability Evaluation Test (2)

The drug sustained releasability of the depot formulation of the present invention was evaluated.

### 4.1 Preparation of Test Article

To a measuring flask, 6 mg of isopropyl (6-{[4-(pyrazol-1-yl)benzyl] (pyridin-3-ylsulfonyl) aminomethyl}pyridin-2-ylamino) acetate (hereinafter also referred to as Compound A) was measured, then dissolved with tri-n-butyl citrate or acetyl tri-n-butyl citrate, diluted to 3 mL total, thereby preparing the formulations of Examples 23 and 24.
In a measuring flask, 6 mg of Compound A was measured, then 0.3 mL of PEG 400 was added, dissolved with tri-n-butyl citrate, and diluted up to 3 mL total, thereby preparing the formulation of Example 25. In a measuring flask, 6 mg of Compound A was measured, then 1 mL of liquid prepared by dissolving 500 mg of sucrose octaacetate with 5 mL of tri-n-butyl citrate was added, and diluted up to 3 mL with tri-n-butyl citrate, thereby preparing the formulation of Example 26.

In standard bottles, 10 mg of compound A was weighed, respectively, dissolved in 5 mL of a mixture of acetyl tri-n-butyl citrate, benzyl benzoate and PEG 400, thereby preparing the formulations of Examples 27 to 30.

In standard bottles, 10 mg of compound A was weighed, dissolved in 5 mL of a mixture of acetyl tri-n-butyl citrate, benzyl benzoate, PEG 400 and vitamin E, thereby preparing the formulation of Example 31.

### 4.2 Preparation of Release Solvent

To a beaker, 800 mL of water for injection, 1 g of polysorbate 80, 3 g of sodium dihydrogen phosphate, and 29 g of dibasic sodium phosphate hydrate were added, then stirred to dissolve. It was diluted in a measuring cylinder to 1 L with water for injection.

### 4.3 Release Test

To a 5-mL standard bottle, 5 mL of release solvent heated to 37°C was added. Using a Hamilton syringe equipped with a 30 G needle, 0.025 mL of the test article was administered, and stirred at 37°C and 86 rpm. One and three, or four, seven, fourteen, twenty-one and twenty-eight days after test article administration, 0.75 mL of release solution was collected, and diluted with 0.75 mL of acetonitrile/water (1:1 by volume ratio). To a standard bottle, 0.75 mL of new release solvent was added. The content of Compound A and tri-n-butyl citrate or acetyl tri-n-butyl citrate in the collected release solvent was measured by HPLC, and the cumulative release rate after administration was calculated.

### 4.4 Test Results and Considerations

The test results are shown in Tables 7 and 8.

**[Table 7]**

| Formula | | Example 23 | Example 24 | Example 25 | Example 26 |
|---|---|---|---|---|---|
| Compound A | | 6 mg | 6 mg | 6 mg | 6 mg |
| Tri-n-butyl citrate | | q.s. | - | q.s. | q.s. |
| Acetyl tri-n-butyl citrate | | - | q.s. | - | - |
| PEG400 | | - | - | 0.3 mL | - |
| Sucrose octaacetate | | - | - | - | 100 mg |
| Total amount | | 3 mL | 3 mL | 3 mL | 3 mL |
| Compound A cumulative release rate (%) | After 1 days | 1.4 | 3.7 | 1.7 | 1.3 |
| | After 4 days | 4.4 | 8.7 | 5.0 | 4.4 |
| | After 7 days | 7.3 | 13.0 | 8.1 | 12.4 |
| Tri-n-butyl citrate or acetyl tri-n-butyl citrate cumulative release rate (%) | After 1 days | 1.5 | 3.2 | 1.2 | 1.5 |
| | After 4 days | 4.6 | 4.4 | 4.3 | 4.4 |
| | After 7 days | 7.3 | 5.5 | 7.5 | 7.1 |

**[Table 8]**

| Formula | | Example 27 | Example 28 | Example 29 | Example 30 | Example 31 |
|---|---|---|---|---|---|---|
| Compound A | | 10 mg | 10 mg | 10 mg | 10 mg | 10 mg |
| Acetyl tri-n-butyl | | 0.5 mL | 1 mL | 1.5 mL | 2 mL | 1.5 mL |
| citrate | | | | | | |
| Benzyl benzoate | | 2 mL | 1.75 mL | 1.5 mL | 1.75 mL | 1.5 mL |
| PEG 400 | | 2.5 mL | 2.25 mL | 2 mL | 1.25 mL | 1.75 mL |
| Vitamin E | | - | - | - | - | 0.25 mL |
| Cumulative release rate of Compound A (%) | After 1 day | 8.0 | 5.8 | 7.3 | 2.8 | 6.5 |
| | After 3 days | 14.7 | 12.8 | 13.9 | 9.0 | 11.3 |
| | After 7 days | 27.2 | 24.7 | 24.2 | 18.6 | 19.3 |
| | After 14 day | 45.5 | 41.6 | 38.6 | 31.8 | 28.5 |
| | After 21 days | 58.0 | 53.2 | 49.0 | 41.1 | 36.0 |
| | After 28 days | 69.0 | 63.8 | 58.1 | 50.5 | 42.4 |

As shown in Table 7, the formulations of Examples 23 to 26 only released 7.3 to 13.0% of Compound A seven days after administration. In addition, tri-n-butyl citrate or acetyl tri-n-butyl citrate, which are bases, were only released in 5.5 to 7.5% seven days after administration. Based on the above, the depot formulation of the present invention was confirmed to sustained-release the drug. Furthermore, as shown in Table 8, the formulations of Examples 27 to 31 prepared at the blending ratios of 10% (v/v) to 40% (v/v) of acetyl tri-n-butyl citrate relative to the total mass of components other than the drug, the cumulative release rate of compound A on the twenty-eighth day after administration was 42.4% to 69.0%, whereby it was confirmed that it is possible to continually release the drug for all blending ratios.

### 5. Sustained Releasability Evaluation Test (3)

The drug sustained releasability of the depot formulation of the present invention was evaluated.

### 5.1 Preparation of Test Article

To 32 mg of 2-[[[2-[(hydroxyacetyl)amino]-4-pyridinyl]methyl]thio]-N-[4-(trifluoromethoxy)phenyl]-3-pyridinecarboxamide (hereinafter also referred to as Compound B), 1 mL of PEG 400 was added, then stirred to dissolve, thereby preparing the formulation of Comparative Example 3.

To 32 mg of Compound B, 0.5 mL of PEG 400 and 0.5 mL of tri-n-butyl citrate were added, then stirred to dissolve, thereby preparing the formulation of Example 32.

To 32 mg of Compound B, 0.5 mL of PEG 400, 0.1 mL of tri-n-butyl citrate and 0.4 mL of acetyl tri-n-butyl citrate were added, then stirred to dissolve, thereby preparing the formulation of Example 33.

To 32 mg of Compound B, 0.5 mL of dimethylsulfoxide and 0.5 mL of tri-n-butyl citrate were added, then stirred to dissolve, thereby preparing the formulation of Example 34.

To 32 mg of Compound B, 0.5 mL of glycofurol and 0.5 mL of tri-n-butyl citrate were added, then stirred to dissolve, thereby preparing the formulation of Example 35.

### 5.2 Preparation of Release Solvent

In a 10-L container, 76.8 g of Dulbecco PBS (-) powder "NISSUI" was measured, and dissolved by adding 8 L of purified water. Furthermore, 8 g of polyethylene glycol monostearate (MYS40) was added, and stirred to dissolve.

### 5.3 Release Test

To a 30-mL standard bottle, 25 mL of release solvent heated to 37°C was added. Using a Hamilton syringe equipped with a 30 G needle, 0.025 mL of the test article was administered, and stirred at 37°C and 86 rpm. One, five and seven days after test article administration, 0.75 mL of release solution was collected, and diluted with 0.75 mL of acetonitrile/water (1:1 by volume ratio). To a standard bottle, 0.75 mL of new release solvent was added. The content of Compound B in the collected release solvent was measured by HPLC, and the cumulative release rate after administration was calculated.

### 5.4 Test Results and Considerations

The test results are shown in Table 9.

**[Table 9]**

| Formula | | Comparati ve Example 3 | Exampl e 32 | Exampl e 33 | Exampl e 34 | Exampl e 35 |
|---|---|---|---|---|---|---|
| Compound B | | 32 mg | 32 mg | 32 mg | 32 mg | 32 mg |
| Tri-n-butyl citrate | | - | 0.5 mL | 0.1 mL | 0.5 mL | 0.5 mL |
| Acetyl tri-n-butyl citrate | | - | - | 0.4 mL | - | - |
| PEG 400 | | 1 mL | 0.5 | 0.5 | - | - |
| | | | mL | mL | | |
| Dimethyl sulfoxide | | - | - | - | 0.5 mL | - |
| Glycofurol | | - | - | - | - | 0.5 mL |
| Compound B cumulative release rate (%) | After 1 days | 15.0 | 12.0 | 6.2 | 7.6 | 6.4 |
| | After 5 days | 32.6 | 17.1 | 9.5 | 10.0 | 11.3 |
| | After 7 days | 50.3 | 18.3 | 10.6 | 18.9 | 12.9 |

As shown in Table 9, the formulation of Comparative Example 3 released at least 50% of Compound B seven days after administration; whereas, the formulations of Examples 32 to 35 only released 10.6 to 18.9% of Compound B seven days after administration. Based on the above, the depot formulation of the present invention was confirmed to controlled release the drug.

### 6. Sustained Releasability Evaluation Test (4)

The drug sustained releasability of the depot formulation of the present invention was evaluated.

### 6.1 Preparation of Test Article

In a standard bottle, 30 mg of INCB28050 was measured, and dissolved by adding 1.5 mL of dimethyl sulfoxide, and 1.5 mL of acetyl triethyl citrate was further mixed, thereby preparing the formulation of Comparative Example 4. In addition, the formulation prepared in Example 10 was used.

### 6.2 Preparation of Release Solvent

To a beaker, 800 mL of water for injection, 1 g of polysorbate 80, 3 g of sodium dihydrogen phosphate, and 29 g of dibasic sodium phosphate hydrate were added, then stirred to dissolve. It was diluted in a measuring cylinder to 1 L with water for injection.

### 6.3 Release Test

To a 5-mL standard bottle, 5 mL of release solvent heated to 37°C was added. Using a Hamilton syringe equipped with a 30 G needle, 0.025 mL of the test article was administered, and stirred at 37°C and 86 rpm. One, three and seven days after test article administration, 0.75 mL of release solution was collected, and diluted with 0.75 mL of acetonitrile/water (1:1 by volume ratio). To a standard bottle, 0.75 mL of new release solvent was added. The content of INCB28050 in the collected release solvent was measured by HPLC, and the cumulative release rate after administration was calculated.

### 6.4 Test Results and Considerations

The test results are shown in Table 10.

**[Table 10]**

| Formula | | Comparative Example 4 | Example 10 |
|---|---|---|---|
| INCB28050 | | 30 mg | 30 mg |
| Tri-n-butyl citrate | | - | 1.5 mL |
| Acetyl tri-n-butyl citrate | | 1.5 mL | - |
| Dimethylsulfoxide | | 1.5 mL | 1.5 mL |
| Cumulative release rate of INCB28050) (%) | After 1 day | 78.1 | 54.6 |
| | After 3 | 91.5 | 80.4 |
| | days | | |
| | After 7 days | 91.6 | 89.6 |

As shown in Table 10, 91.5% of the administered amount of INCB28050 was released by the formulation of Comparative Example 4 three days after administration; whereas, only 89.6% of the administered amount of INCB28050 was released seven days after administration by the formulation of Example 10, and thus it was confirmed that the sustained-releasability was improved by the present depot formulation.

### 7. Sustained Releasability Evaluation Test (5)

The drug sustained releasability of the depot formulation of the present invention was evaluated.

### 7.1 Preparation of Test Article

In a standard bottle, 30 mg of Ciclosporin A was measured, and dissolved by adding 3 mL of acetyl triethyl citrate and mixing, thereby preparing the formulation of Comparative Example 5. In addition, the formulations prepared in Examples 11 and 12 were used.

### 7.2 Preparation of Release Solvent

To a beaker, 800 mL of water for injection, 1 g of polysorbate 80, 3 g of sodium dihydrogen phosphate, and 29 g of dibasic sodium phosphate hydrate were added, then stirred to dissolve. It was diluted in a measuring cylinder to 1 L with water for injection.

### 7.3 Release Test

To a 5-mL standard bottle, 5 mL of release solvent heated to 37°C was added. Using a Hamilton syringe equipped with a 30 G needle, 0.025 mL of the test article was administered, and stirred at 37°C and 86 rpm. One, three and seven days after test article administration, 0.75 mL of release solution was collected, and diluted with 0.75 mL of acetonitrile/water (1:1 by volume ratio). To a standard bottle, 0.75 mL of new release solvent was added. The content of Ciclosporin A in the collected release solvent was measured by HPLC, and the cumulative release rate after administration was calculated.

### 7.4 Test Results and Considerations

The test results are shown in Table 11.

**[Table 11]**

| Formula | | Comparative Example 5 | Example 11 | Example 12 |
|---|---|---|---|---|
| Ciclosporin A | | 30 mg | 30 mg | 30 mg |
| Acetyl tri-n-butyl citrate | | - | 3 mL | - |
| Tri-n-butyl citrate | | - | - | 3 mL |
| Acetyl triethyl citrate | | 3 mL | - | - |
| Cumulative release rate of Ciclosporin A (%) | After 1 day | 5.8 | 6.0 | 5.4 |
| | After 3 days | 13.4 | 13.2 | 12.4 |
| | After 7 days | 22.3 | 21.3 | 20.0 |
| | After 14 days | 33.8 | 29.7 | 28.4 |
| | After 21 | 51.0 | 38.0 | 36.9 |
| | days | | | |
| | After 28 days | 66.3 | 46.2 | 45.6 |

As shown in Table 11, 66.3% of the administered amount of Ciclosporin A was released by the formulation of Comparative Example 5 twenty-eight days after administration; whereas, only 46.2% and 45.6% of the administered amount of Ciclosporin A was released by the formulations of Examples 11 and 12, respectively, twenty-eight days after administration, and thus it was confirmed that the sustained-releasability was improved by the present depot formulation.

### 8. Sustained Releasability Evaluation Test (6)

The drug sustained releasability in an animal of the depot formulation of the present invention was evaluated.

### 8.1 Preparation of Test Article

In standard bottles, 240 mg of sirolimus was weighed, and after dissolving by adding 0.8 mL of dimethyl sulfoxide, 7.2 mL of acetyl triethyl citrate and acetyl tri-n-butyl citrate and mixing, followed by performing filtration sterilization with a filter of 0.20 µm pore size, thereby preparing the formulations of Comparative Example 6 and Example 36.

In standard bottles, 240 mg of sirolimus was weighed, and after dissolving by adding 3.6 mL of benzyl benzoate/ethanol (40:5 by volume ratio) or 3.68 mL of Vitamin E/benzyl benzoate/ethanol (1:40:5 by volume ratio) that had been mixed in advance, 4.4 mL or 4.32 mL of acetyl tri-n-butyl citrate was added and mixed, followed by performing filtration sterilization with a filter of 0.20 µm pore size, thereby preparing the formulations of Example 37 and Example 38.

### 8.2 Rabbit Pharmacokinetics Evaluation

Using a Hamilton syringe equipped with a 30G needle, 0.03 mL of the depot formulations of Comparative Example 6 and Examples 36 to 38 per eye of albino rabbit was intravitreally administered. After four weeks and after twelve weeks from administration, euthanization was conducted with anesthetic by intravenous administration of pentobarbital sodium, and the eyeballs were enucleated. The enucleated eyeballs were immediately frozen, and the vitreous body was collected in a state containing the depot formulation. The sirolimus concentration in the vitreous body at each time point of collection was measured using a LC-MS/MS, and the drug residual amount after administration was evaluated.

### 8-3. Test Results and Considerations

The test results are shown in Table 12.

**[Table 12]**

| Formula | | Comparative Example 6 | Example 36 | Example 37 | Example 38 |
|---|---|---|---|---|---|
| sirolimus | | 240 mg | 240 mg | 240 mg | 240 mg |
| Acetyl tri-n-butyl citrate | | - | 7.2 mL | 4.4 mL | 4.32 mL |
| Acetyl triethyl citrate | | 7.2 mL | - | - | - |
| Benzyl benzoate | | - | - | 3.2 mL | 3.2 mL |
| Dimethylsulfoxide | | 0.8 mL | 0.8 mL | | |
| Ethanol | | - | - | 0.4 mL | 0.4 mL |
| Vitamin E | | - | - | - | 0.08 mL |
| Residual rate of sirolimus (%) | After 4 weeks | 10.0 | 67.3 | 71.1 | 83.7 |
| | After 12 weeks | 0.2 | 32.5 | 29.4 | 73.8 |

As shown in Table 12, only 10.0% of the administered amount of sirolimus remained at four weeks after administration for the formulation of Comparative Example 6; whereas, 67.3% of the administered amount for the formulation of Example 36, 71.1% of Example 37 and 83.7% of Example 38 remained.
According to the above results, it was confirmed that the sustained-releasability was improved by the present depot formulation.

### 9. Sustained Releasability Evaluation Test (7)

The drug sustained-releasability in an animal of the depot formulation of the present invention was evaluated.

### 9-1. Preparation of Test Article

In a standard bottle, 15 mg of latanoprost was weighed, and after adding 3 mL of acetyl triethyl citrate and acetyl tri-n-butyl citrate to dissolve and mixing, filter sterilization was performed with a filter 0.20 µm pore size, thereby preparing the formulations of Comparative Example 7 and Example 39.

### 9-2. Rabbit Pharmacokinetic Evaluation

Using a Hamilton syringe equipped with a 30 G needle, the depot formulations of Comparative Example 7 and Example 39 were intravitreally administered at 0.02 mL per eye of albino rabbit, respectively. After two weeks and after four weeks from administration, euthanization was conducted with anesthetic by intravenous administration of pentobarbital sodium, and the eyeballs were enucleated. The enucleated eyeballs were immediately frozen, and the vitreous body was collected in a state containing the depot formulation. The latanoprost concentration in the each vitreous body at the time point of collection was measured using a LC-MS/MS, and the drug residual amount after administration was evaluated. In addition, the carboxylate-form latanoprost concentration in the iris-ciliary body was measured using an LC-MS/MS.

### 9.3 Test Results and Considerations

The test results are shown in Table 13.

**[Table 13]**

| Formula | | Comparative Example 7 | Example 39 |
|---|---|---|---|
| Latanoprost | | 15 mg | 15 mg |
| Acetyl tri-n-butyl citrate | | - | 3 mL |
| Acetyl triethyl citrate | | 3 mL | - |
| Residual rate of latanoprost (%) | After 2 weeks | <0.3 | 13.0 |
| | After 4 weeks | <0.3 | 4.8 |
| carboxylate-form latanoprost concentration in the iris-ciliary body (ng/g) | After 2 weeks | <10.3 | 57.1 |
| | After 4 weeks | <10.3 | 21.3 |

As shown in Table 13, the latanoprost residual rate was less than 0.3% at two weeks after administration; whereas, 13.0% remained for the formulation of Example 39. In addition, in the iris-ciliary body that was the target tissue, the carboxylate-form latanoprost concentration, which is the active substance, was less than 10.3 ng/g at two weeks after administration for Comparative Example 7; whereas, it was 57.1 ng/g for the formulation of Example 39, and thus a drug concentration of a sufficient amount was measured even after four weeks from administration. According to the above results, it was confirmed that the sustained-releasability was improved by the present depot formulation.

### 10. Sustained Releasability Evaluation Test (8)

The drug sustained releasability in an animal of the depot formulation of the present invention was evaluated.

### 10.1 Preparation of Test Article

In standard bottles, 15 mg of Compound A was weighed, and after dissolving by adding 3 mL of acetyl triethyl citrate and acetyl tri-n-butyl citrate and mixing, filtration sterilization with was performed with a filter of 0.20 µm pore size, thereby preparing the formulations of Comparative Example 8 and Example 40.

In standard bottles, 15 mg of Compound A was weighed, and after dissolving by adding 3 mL of acetyl tri-n-butyl citrate, benzyl benzoate and PEG 400 and mixing, filtration sterilization with was performed a filter of 0.20 µm pore size, thereby preparing the formulations of Example 41 and Example 42.

### 10.2 Rabbit Pharmacokinetic Evaluation

Using a Hamilton syringe equipped with a 30 G needle, the depot formulations of Comparative Example 8, Example 40, Example 41 and Example 42 were intravitreally administered at 0.02 mL per eye of albino rabbit, respectively. For Comparative Example 8 and Example 40, after four weeks and after twelve weeks from administration, and for Examples 41 and 42, after four weeks from administration, euthanization was conducted with anesthetic by intravenous administration of pentobarbital sodium, and the eyeballs were enucleated. The enucleated eyeballs were immediately frozen, and the vitreous body was collected in a state containing the depot formulation. The Compound A concentration in each vitreous body at the time point of collection was measured using a LC-MS/MS, and the drug residual amount after administration was evaluated. In addition, the carboxylate-form Compound A concentration in the iris-ciliary body was measured using an LC-MS/MS.

### 10.3 Test Results and Considerations

The test results are shown in Table 14.

**[Table 14]**

| Formula | | Comparative Example 8 | Example 40 | Example 41 | Example 42 |
|---|---|---|---|---|---|
| Compound A | | 15 mg | 15 mg | 15 mg | 15 mg |
| Acetyl tri-n-butyl citrate | | - | 3 mL | 1.5 mL | 0.9 mL |
| Acetyl tri-n-ethyl citrate | | 3 mL | - | - | - |
| Benzyl benzoate | | - | - | 1.35 mL | 0.9 mL |
| PEG 400 | | - | - | 0.15 mL | 1.2 mL |
| Residual rate of Compound A (%) | After 4 weeks | 17.0 | 74.8 | 79.1 | 75.3 |
| | After 12 weeks | 11.3 | 33.4 | - | - |
| carboxylate-form Compound A concentration in the iris-ciliary body (ng/g) | After 4 weeks | 20.0 | 114 | 78.2 | 68.3 |
| | After 12 weeks | 2.19 | 17.4 | - | - |

As shown in FIG. 14, the Compound A residual rate was less than 17.0% at four weeks after administration for the formulation of Comparative Example 8; whereas, 74.8% to 79.1% remained for the formulations of Examples 40 to 42. In addition, in the iris-ciliary body that was the target tissue, the carboxylate-form Compound A concentration, which is the active substance, was less than 20.0 ng/g at four weeks after administration for the formulation of Comparative Example 8; whereas, it was 68.3 to 114 ng/g for the formulations of Examples 40 to 42. Furthermore, these were results in which Example 40 exhibited more sustained releasability than Comparative Example 8 even after twelve weeks from administration. In addition, the same extent of sustained releasability was exhibited with the blending ratios of acetyl tri-n-butyl citrate relative to the total amount of components other than drug of 30% (v/v) to 100% (v/v), and thus it was confirmed to be possible to set the blending ratio according to the purpose. According to the above results, it was confirmed that the sustained-releasability was improved by the present depot formulation.

### 11. Safety Evaluation Test

The tolerability of the depot formulation of the present invention was evaluated.

### 11.1 Preparation of Test Article

The formulations of Comparative Example 9 and Example 43 were prepared by performing filtration sterilization with a filter of 0.20 µm pore size on acetyl triethyl citrate and acetyl tri-n-butyl citrate.

The formulation of Example 44 was prepared by performing filtration sterilization with a filter of 0.20 µm pore size on acetyl tri-n-butyl citrate.

### 11.2 Histopathology Evaluation

Using a Hamilton syringe equipped with a 30G needle, 0.05 mL of the formulation of Comparative Example 9 and Example 43 per eye of albino rabbit was intravenously administered. After two month intravitreal administration of the test article, rabbits were anesthetized by intravenous administration of pentobarbital sodium, and euthanized by exsanguination. The eyeball was enucleated and fixed in the F-G fixative (a mixture of 10% neutral buffered formalin and 2.5% glutaraldehyde) for 24 hours, followed by re-fixation in 10% neutral buffered formalin. Paraffin embedding was performing following a standard method, and after slicing to reveal a section was completed, intermittent serial sections at 1 mm intervals were prepared from the side of the nose to the side of the ears in the sagittal plane. After preparation of paraffin sections by a standard method, microscopic examination was performed after conducting HE staining.

For the formulation of Example 44, except for performing euthanization by exsanguination one month after administration instead of euthanization by exsanguination two months after administration, sections were prepared and examined under microscope similarly to as described above.

### 11.3 Test Results and Considerations

The test results are shown in FIG. 2, FIG. 3, FIG. 4 and Table 15.

**[Table 15]**

| Region: observation | Preparation | Comparative Example 9 | Example 43 |
|---|---|---|---|
| | Dosage | 50 µL/eye | 50 µL/eye |
| | Test case number | 6 | 6 |
| Macroscopic findings | grade*: | | |
| Vitreous body: transparent | P | 0** | 6 |
| Vitreous body: white substance | P | 0 | 0 |
| Vitreous body: cloudv | P | 0 | 0 |
| Retina: peeling | P | 0 | 0 |
| Histological findings | grade*: | | |
| Retina: localized necrosis | +++ | 4** | 0 |
| Retina: inflammatory cell permeation | ± | 0 | 0 |
| | + | 0 | 0 |
| Retina: peeling | +++ | 0 | 0 |
| Vitreous body: basophil substance | P | 1 | 1 |
| Vitreous body: granuloma | + | 0 | 0 |
| | ++ | 0 | 0 |
| | +++ | 0 | 0 |
| Vitreous body: inflammatory cell permeation | + | 0 | 0 |
| Choroid coat: inflammatory cell permeation | + | 0 | 0 |
| Ciliary body: inflammatory cell permeation | + | 0 | 0 |
| | ++ | 0 | 0 |
| Crystalline lens: denaturing of fiber | + | 0 | 0 |
| Optic papilla: denaturing | +++ | 0 | 0 |

| | | | |
|---|---|---|---|
| *:Grade (P = findings available, ±= variation of very slight degree, + = variation of slight degree, ++ = moderate variation, +++ = severe variation) **:Number of cases expressed | | | |

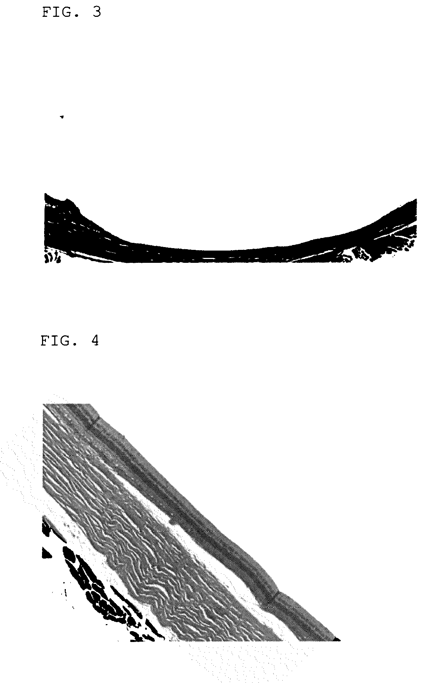

FIGS. 2 and 3 show microscope images of the retina in the vicinity of formulations of Comparative Example 9 and Example 43, respectively (FIG. 2 is 2.5x object, and FIG. 3 is 10x). FIG. 2A is the retina at the underside of the eyeball, and the region indicated by the arrow in the figure is recognized as having thinned. FIG. 2B is a magnified view of FIG. 2A, whereby it is recognized that the entire layer of retina underwent necrosis, and a part thereof calcified.

Macroscopic and histological findings are shown in Table 15. In the macroscopic findings, a depot that is a transparent substance was observed for Example 43; whereas, a depot could not be confirmed for Comparative Example 9. In addition, in the histological findings, although the toxicity findings in the ocular tissue tended to be the most susceptible to the influence of the test article in Example 43 (granuloma and inflammatory cell permeation in vitreous body, localized necrosis in retina, etc.), localized necrosis was recognized in the retina for Comparative Example 9.

Based on the above results, it was confirmed that the present depot formulation is a base that can be safely used.

FIG. 4 provides microscope photographs (10x object) of retina in the vicinity of the test article of Example 44, whereby toxicity finding (granulation containing administered matter and inflammatory cell permeation in the vitreous body, atrophy/loss of retina and dysplasia, etc.) in the ocular tissue that is the most susceptible to the influence of the test article was not observed. In addition, toxicity finding (inflammatory cell permeation at angulus iridocornealis periphery and swelling/inflammatory cell permeation in corneal stroma, etc.) was not observed in other ocular tissue. Based on the above results, it was confirmed that the depot formulation can be safely used for a vehicle.

## Claims

1. A depot formulation comprising a trialkyl citrate and/or acetyl trialkyl citrate, wherein alkyl groups possessed by each of the trialkyl citrate and the acetyl trialkyl citrate are the same or different, and have a number of carbon atoms of 3 to 5.

2. The depot formulation according to claim 1, wherein the number of carbon atoms of the alkyl group is 4.

3. The depot formulation according to claim 1, wherein the trialkyl citrate is tri-n-butyl citrate, and the acetyl trialkyl citrate is acetyl tri-n-butyl citrate.

4. The depot formulation according to any one of claims 1 to 3, further comprising a drug.

5. The depot formulation according to claim 4, wherein the drug is a compound represented by formula (1) or a salt thereof, 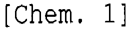 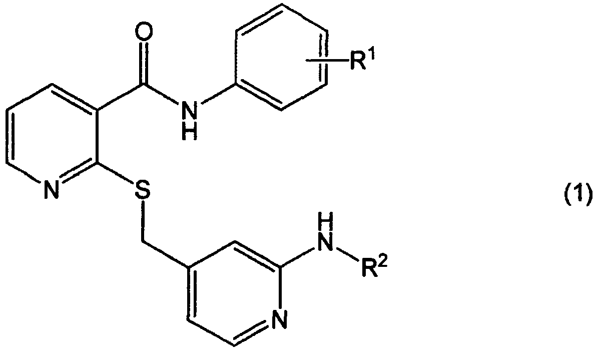 wherein
R¹ denotes a hydrogen atom, halogen atom, hydroxyl group, C₁₋₆ alkyl group, C₁₋₆ alkyl group substituted by one or a plurality of halogen atoms, C₁₋₆ alkoxy group or C₁₋₆ alkoxy group substituted by one or a plurality of halogen atoms; and
R² denotes a hydrogen atom, C₁₋₆ alkyl group, C₁₋₆ alkylcarbonyl group or C₁₋₆ alkylcarbonyl group substituted by one or a plurality of hydroxyl groups.

6. The depot formulation according to claim 5, wherein the compound represented by formula (1) is 2-[[[2-[(hydroxyacetyl)amino]-4-pyridinyl]methyl]thio]-N-[4-(trifluoromethoxy)phenyl]-3-pyridinecarboxamide or a salt thereof.

7. The depot formulation according to claim 4, wherein the drug is isopropyl (6-{[4-(pyrazol-1-yl)benzyl](pyridin-3-ylsulfonyl)aminomethyl}pyridin-2-ylamino)acetate or a salt thereof.

8. The depot formulation according to claim 4, wherein the drug is nepafenac, dexamethasone, indomethacin, diclofenac sodium, levofloxacin, INCB28050, ciclosporin A, timolol maleate, fluocinolone acetonide, triamcinolone acetonide, budesonide, olopatadine, latanoprost, isopropyl (6-{[4-(pyrazol-1-yl)benzyl](pyridin-3-ylsulfonyl)aminomethyl}pyridin-2-ylamino)acetate, 2-[[[2-[(hydroxyacetyl)amino]-4-pyridinyl]methyl]thio]-N-[4-(trifluoromethoxy)phenyl]-3-pyridinecarboxamide, or sirolimus.

9. The depot formulation according to any one of claims 4 to 8, comprising 0.001 to 30% (w/w) of the drug.

10. The depot formulation according to any one of claims 1 to 8, comprising at least 0.1% (w/w) of the trialkyl citrate and/or acetyl trialkyl citrate.

11. The depot formulation according to any one of claims 1 to 10, wherein the depot formulation is for ocular topical administration.

12. The depot formulation according to claim 11, wherein the depot formulation is for intravitreal administration, subconjunctival administration or intracameral administration.

13. The depot formulation according to any one of claims 1 to 12, wherein the depot formulation is for the prevention and/or treatment of eye disease.

14. A method for forming a depot, comprising bringing a liquid composition containing a trialkyl citrate and/or an acetyl trialkyl citrate into contact with water, a phosphate buffer solution, body fluid or simulated body fluid,
wherein alkyl groups possessed by each of the trialkyl citrate and the acetyl trialkyl citrate are the same or different, and have a number of carbon atoms of 3 to 5.
